(19) Europäisches Patentamt
European Patent Office
Office européen des brevets

(11) **EP 3 477 234 A1**

(12) **EUROPÄISCHE PATENTANMELDUNG**

(43) Veröffentlichungstag:
**01.05.2019 Patentblatt 2019/18**

(51) Int Cl.:
**F25D 31/00** *(2006.01)* **F25B 21/02** *(2006.01)*
**C12M 1/00** *(2006.01)* **B01L 7/00** *(2006.01)*

(21) Anmeldenummer: **18201990.1**

(22) Anmeldetag: **23.10.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **24.10.2017 DE 102017124883**
**07.12.2017 DE 102017129119**

(71) Anmelder: **Liebherr-Hausgeräte Lienz GmbH**
**9900 Lienz (AT)**

(72) Erfinder: **ZOIER, Hans**
**9907 Tristach (AT)**

(74) Vertreter: **Herrmann, Uwe**
**Lorenz Seidler Gossel**
**Rechtsanwälte Patentanwälte**
**Partnerschaft mbB**
**Widenmayerstraße 23**
**80538 München (DE)**

(54) **TEMPERIERTES GERÄT**

(57) Die vorliegende Erfindung betrifft ein temperiertes Gerät, insbesondere Kühl- und/oder Gefriergerät, mit einem Gerätekorpus (10) und mit einem Verschlusselement (20), mittels dessen der temperierte Innenraum (100) des Gerätes verschließbar ist, wobei in dem temperierten Innenraum wenigstens ein erster und ein zweiter Temperaturfühler (T1,Tn) zur Erfassung eines ersten und eines zweiten Temperaturwertes angeordnet sind, die sich in unterschiedlichen Höhenpositionen befinden und wobei ein Regler vorgesehen ist, der ausgebildet ist, den Istwert der Temperaturdifferenz zwischen den beiden Temperaturwerten mit einem Sollwert zu vergleichen, wobei in einem oberen Bereich und/oder in einem relativ dazu unten angeordneten Bereich des Gerätes mindestens ein Peltierelement angeordnet ist, das mit dem Regler in Verbindung steht, wobei der Regler derart ausgebildet ist, dass der Regler das oder die Peltierelemente so ansteuert, dass die Abweichung zwischen Ist- und Sollwert gleich Null ist oder einen Grenzwert nicht übersteigt.

Fig. 1

EP 3 477 234 A1

**Beschreibung**

Die vorliegende Erfindung betrifft ein temperiertes Gerät, insbesondere ein Kühl- und/oder Gefriergerät, mit einem Gerätekorpus und einem Verschlusselement, mittels dessen der temperierte Innenraum des Gerätes verschließbar ist.

In temperierten Geräten, d.h. in Geräten, die einen temperierten Innenraum aufweisen, wie z.B. bei Kühl-, Gefrier- oder Heizgeräten, bildet sich aus physikalischen Gründen üblicherweise eine Temperaturschichtung, d.h. ein Temperaturgradient in vertikaler Richtung in dem gekühlten bzw. beheizten Innenraum aus.

Bei dynamischen Geräten, bei denen in dem Innenraum eine Luftumwälzung durch einen Ventilator erfolgt, ist diese Temperaturschichtung üblicherweise geringer als bei statischen Geräten, bei denen eine solche erzwungene Konvektion nicht erfolgt. Die dynamische Luftumwälzung, d.h. die Luftbewegung mittels eines Ventilators bringt gegenüber statischen Geräten aufgrund des Betriebs des Ventilators allerdings eine energetische und geräuschtechnische Verschlechterung des Gerätes mit sich.

Bei vielen Lageranwendungen in Temperierschränken wird eine möglichst geringe Temperaturschichtung gefordert.

Der vorliegenden Erfindung liegt die Aufgabe zugrunde, ein temperiertes Gerät der eingangs genannten Art dahingehend weiterzubilden, dass die Temperaturschichtung in dem temperierten Innenraum so gering wie möglich ist.

Diese Aufgabe wird durch ein temperiertes Gerät mit den Merkmalen des Anspruchs 1 gelöst.

Danach ist vorgesehen, dass in dem temperierten Innenraum wenigstens ein erster und zweiter Temperaturfühler zur Erfassung eines ersten und eines zweiten Temperaturwertes angeordnet sind, die sich in unterschiedlichen Höhenpositionen des Innenraums befinden und dass ein Regler vorgesehen ist, der ausgebildet ist, die Differenz aus den durch die Temperaturfühler erfassten Temperaturwerten, d.h. den Istwert der Temperaturdifferenz mit einem Sollwert für die Temperaturdifferenz zu vergleichen.

Des Weiteren ist in einem oben angeordneten Bereich und/oder in einem relativ dazu unten angeordneten Bereich des Gerätes mindestens ein Peltierelement angeordnet, das mit dem Regler in Verbindung steht, wobei der Regler derart ausgebildet ist, dass er das oder die Peltierelemente so ansteuert, dass die Abweichung zwischen Ist- und Sollwert der Temperaturdifferenz gleich Null ist oder einen Grenzwert nicht übersteigt.

Der Sollwert der Temperaturdifferenz kann fest vorgegeben oder werkseitig oder auch durch einen Nutzer einstellbar sein. Dieser Sollwert wird je nach dem tolerierten bzw. gewünschten Temperaturunterschied zwischen den beiden Temperaturfühlern vorgegeben bzw. eingestellt. Ist überhaupt keine Temperaturschichtung bzw. - differenz gewünscht, beträgt der Sollwert Null.

Der Regler steuert das oder die Peltierelemente so an, dass der Istwert der Temperaturdifferenz vollständig oder möglichst weitgehend dem Sollwert entspricht.

Das oder die Peltierelemente können je nach Bedarf so angeordnet bzw. betrieben werden, dass diese im Betrieb den temperierten Innenraum kühlen oder heizen.

Ein Peltierelemente kann im unteren Drittel des Gerätes in Höhenrichtung angeordnet sein und/oder ein Peltierelemente kann im oberen Drittel des Gerätes in Höhenrichtung angeordnet sein.

Vorzugsweise ist ein Peltierelemente im Geräteboden angeordnet.

Alternativ oder zusätzlich kann ein Peltierelemente in der Gerätedecke angeordnet sein.

Bei dem temperierten Gerät kann es sich um ein Kühl- und/oder Gefriergerät handeln, wobei der Regler vorzugsweise derart ausgebildet ist, dass das im oberen Bereich angeordnete Peltierelement aktiviert wird, d.h. kühlt oder dessen Kühlleistung erhöht wird, wenn die Differenz zwischen Temperatur-Ist- und Sollwert einen Grenzwert übersteigt, d.h. die Temperaturschichtung innerhalb des gekühlten Innenraums zu groß wird.

Bei dem temperierten Gerät kann es sich auch um beheiztes Gerät, insbesondere um einen Wärmeschrank handeln und der Regler kann derart ausgebildet sein, dass das im unteren Bereich angeordnete Peltierelement aktiviert wird, d.h. heizt oder dessen Heizleistung erhöht wird, wenn die Differenz zwischen Ist- und Sollwert der Temperaturdifferenzen einen Grenzwert übersteigt.

Auch ist der Fall denkbar, dass in dem Gerät sowohl oben als auch unten Peltierelemente angeordnet sind.

Grundsätzlich können das oder die Peltierelemente vollständig oder teilweise innerhalb des Gerätekorpus bzw. innerhalb von dessen Wärmedämmung angeordnet sein. Sind sie teilweise innerhalb des Gerätekorpus bzw. innerhalb der Wärmedämmung angeordnet, können sie mit ihrer kalten oder warmen Seite in den temperierten Innenraum hineinragen. Dies gilt für etwaige Wärmeübertragungsflächen, die mit dem Peltierelement in wärmeleitender Verbindung stehen, entsprechend.

Handelt es sich um ein Kühl- bzw. Gefriergerät, kann vorgesehen sein, die Leistung des unten angeordneten Peltierelementes zu reduzieren, so dass dieses weniger stark kühlt und/oder die Leistung des oben angeordneten Peltierelementes zu steigern so dass dieses stärker kühlt. Beides wirkt der nicht gewünschten Temperaturschichtung (unten kalt - oben warm) entgegen.

Handelt es sich um ein beheiztes Gerät, kann vorgesehen sein, die Leistung des unten angeordneten Peltierelementes zu steigern, so dass der Wärmeeintrag größer wird und/oder die Leistung es oben angeordneten Peltierelementes zu reduzieren, so dass dessen Wärmeeintrag verringert wird. Beides wirkt der nicht gewünschten Temperaturschichtung (unten kalt - oben warm) entgegen.

Es kann sich bei dem temperierten Gerät grundsätzlich um ein Kühl- und/oder Gefriergerät handeln, d.h. um ein Gerät in dessen gekühlten Innenraum eine Temperatur herrscht, die geringer ist als die Umgebungstemperatur (z.B. 20 °C). Es kann sich wie ausgeführt jedoch auch um ein beheiztes Gerät handeln, wie z.B. um einen Wärmeschrank, bei dem ein Wärmeeintrag mittels Peltierelement in den Innenraum gewünscht ist, um ein Temperaturniveau über Zimmertemperatur zu erzielen.

Denkbar ist es, dass außer den beiden genannten Peltierelementen keine weiteren Vorrichtungen zur Kühlung und/oder Erwärmung des gekühlten Innenraums vorgesehen sind.

Von der Erfindung ist jedoch auch der Fall umfasst, dass außer den beiden genannten Peltierelementen weitere Vorrichtungen zur Kühlung und/oder Erwärmung des gekühlten Innenraums vorgesehen sind, wie z.B. weitere Peltierelemenete oder auch ein herkömmlicher Kältemittelkreislauf.

Der Regler kann so ausgeführt sein, dass dieser aus den durch die Temperaturfühler erfassten Werten einen Mittelwert bildet und diesen mit einer Solltemperatur vergleicht, die in dem gekühlten Innenraum herrschen soll. Auch ist es denkbar, dass der nur von einem Temperaturfühler erfasste Wert als Istwert zum Zwecke der Temperaturregelung herangezogen wird.

Basierend auf der Differenz zwischen dem Mittelwert mehrerer gemessener Temperaturwerte und der Solltemperatur nimmt der Regler durch Ansteuerung des oder der Peltierelemente oder sonstiger Heiz- und/oder Kühlmittel die Temperaturregelung des temperierten Innenraums vor. Der Regler hat in diesem Fall nicht nur die Aufgabe, ein bestimmtes mittleres Temperaturniveau innerhalb des temperierten Innenraums einzustellen, sondern zusätzlich die Aufgabe, eine Temperaturschichtung zu verhindern oder unter einem Grenzwert zu halten.

Um einen möglichst großen Eintrag oder eine möglichst große Abfuhr von Wärme in den bzw. aus dem temperierten Innenraum zu erzielen, kann das oder können die Peltierelemente mit einem Wärmetauscher in Verbindung stehen, der seinerseits mit dem temperierten Innenraum in Verbindung steht, um eine möglichst große Wärmeübertragungsfläche bereitzustellen. Der Wärmetauscher bzw. Wärmeübertrager kann beispielsweise einen Teil des Innenbehälters darstellen, der den temperierten Innenraum zusammen mit der Tür oder einem sonstigen Verschlusselement begrenzt.

An dieser Stelle wird darauf hingewiesen, dass die Begriffe "ein" und "eine" nicht zwingend auf genau eines der Elemente verweisen, wenngleich dies eine mögliche Ausführung darstellt, sondern auch eine Mehrzahl der Elemente bezeichnen können. Ebenso schließt die Verwendung des Plurals auch das Vorhandensein des fraglichen Elementes in der Einzahl ein und umgekehrt umfasst der Singular auch mehrere der fraglichen Elemente. Es können somit ein, zwei oder mehr als zwei Peltierelemente vorhanden sein. Auch ist es denkbar, genau zwei Temperaturfühler oder auch mehr als zwei Temperaturfühler einzusetzen.

Weitere Einzelheiten und Vorteile der Erfindung werden anhand eines in der Zeichnung dargestellten Ausführungsbeispiels näher erläutert.

Es zeigen:

Figur 1:   eine schematische Längsschnittansicht durch ein Gerät gemäß der Erfindung,

Figur 2:   eine mögliche Schaltungsvariante zur Verschaltung der Peltierelemente sowie eine schematische Darstellung des Regelungskreises.

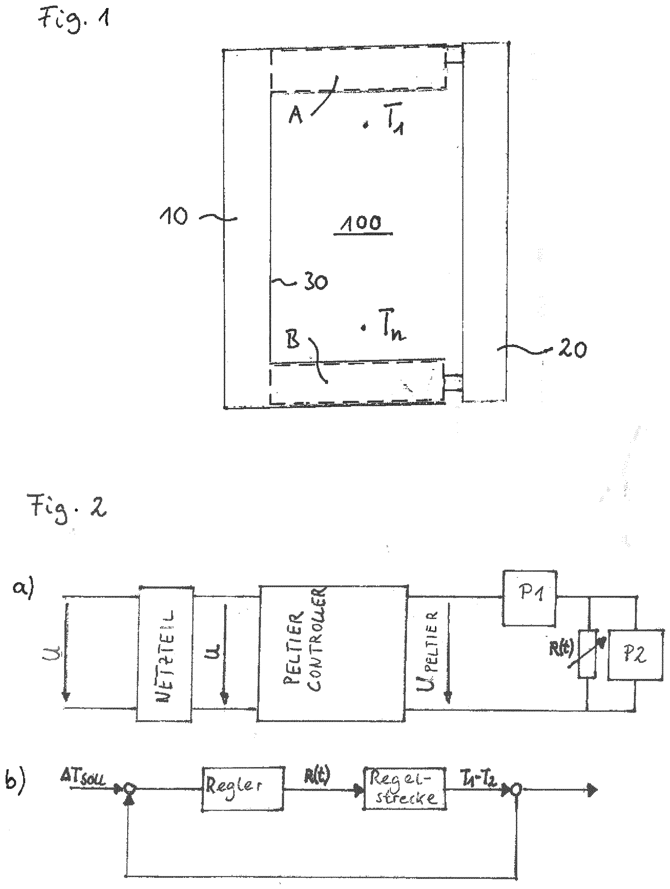

Figur 1 zeigt mit dem Bezugszeichen 10 den wärmeisolierten Korpus eines schrankartigen Kühl- und/oder Gefriergerätes gemäß der Erfindung.

Mit 20 ist die Tür dargestellt, die den gekühlten Innenraum 100 zusammen mit dem Innenbehälter 30 begrenzt.

Der gestrichelte Bereich A kennzeichnet die Gerätedecke und der gestrichelte Bereich B kennzeichnet den Geräteboden des Gerätekorpus.

Wie auch die Rückwand und die beiden Seitenwände sind diese wärmedämmend ausgeführt (z.B. PU-Schaum, Vakuum etc.). Dies gilt für die Tür entsprechend.

Innerhalb der Bereiche A und B befinden sich je ein oder mehrere Peltierelemente mit Wärmetauscherkomponenten zur Einstellung bzw. Verhinderung der Temperaturschichtung innerhalb des gekühlten Innenraums 100. Je nach Ihrer Anordnung beheizen oder kühlen die Peltierelemente den Innenraum 100.

Dabei sind die Peltierelemente bzw. deren Wärmetauscherkomponenten je nach Anwendung (Kühl- und/oder Gefriergerät oder Wärmegerät) so angeordnet, dass deren kalte oder deren warme Seite mit dem Innenraum 100 in wärmeübertragendem Kontakt steht, so dass dieser mittels der Peltierelemente beheizbar bzw. kühlbar ist.

Die Temperaturerfassung in dem gekühlten Innenraum 100 erfolgt über zumindest zwei Temperaturfühler T1 und Tn, die die Temperaturschichtung in vertikaler Richtung und die Innenraumtemperatur für die Regelung als Funktion eines oder mehrerer Temperaturfühlerwerte wiedergeben.

Wird der Innenraum 100 gekühlt, ist gemäß dem Ausführungsbeispiel zumindest in der Gerätedecke A ein Peltierelement angeordnet, das dem Innenraum Wärme entzieht und das zur Steuerung der Temperaturschichtung herangezogen werden kann. Die notwendige Kälteleistung für das Gerät wird vorzugsweise über zumindest ein zusätzliches Peltierelement erzeugt.

Wird der Innenraum 100 beheizt, muss zumindest in dem Geräteboden B ein Peltierelement angeordnet sein, das dem Innenraum Wärme zuführt und das zur Steuerung der Temperaturschichtung herangezogen werden kann. Die notwendige Heizleistung für das Gerät wird vorzugsweise über zumindest ein zusätzliches Peltierelement erzeugt.

Sind in der Gerätedecke A und in dem Geräteboden B Peltierelemente verbaut, so kann im Heiz- und im Kühlfall die Temperaturschichtung besonders wirksam minimiert werden.

Die notwendige Heiz- oder Kälteleistung kann über zumindest ein zusätzliches Peltierelement erzeugt werden, ja nach dem Leistungsbedarf können aber auch die Peltierelemente in den Bereichen A und B ausreichen, um das gewünschte Temperaturniveau in dem gekühlten/beheizten Innenraum einzustellen.

Zur Steuerung bzw. Beseitigung der Temperaturschichtung in dem gekühlten Innenraum wird nun die Leistung am Peltierelement im Deckenbereich A bzw. in Bodenbereich B verändert. Bei einem gekühlten Innenraum wird bei Feststellung einer unerwünschten Temperaturschichtung (unten kalt - oben warm) die Leistung von dem im Bereich B befindlichen Element verringert (weniger Kälteleistung) und/oder die Leistung von dem im Bereich A befindlichen Element erhöht (mehr Kälteleistung).

Bei einem beheizten Innenraum wird die Leistung von dem im Bereich B befindlichen Element erhöht (mehr Wärmeeintrag) und/oder die Leistung von dem im Bereich A befindlichen Element verringert (weniger Wärmeeintrag).

Wie dies aus Figur 1 hervorgeht, befinden sich die Temperaturfühler in vertikaler Richtung voneinander beabstandet in dem gekühlten Innenraum, wobei der Temperaturfühler $T_1$ weiter oben angeordnet ist als der Temperaturfühler $T_n$.

Der als Regelgröße herangezogene Temperaturistwert $T_{ist}$ ist eine Funktion der gemessenen Temperaturistwerte der einzelnen Temperaturfühler, d.h.

$$T_{ist} = f\,(T_1,\ ....,\ T_n).$$

Eine denkbare Abhängigkeit wäre der arithmetische Mittelwert, d.h.

$$T_{ist} = \sum_{i=1}^{n} T_i\,/\,n$$

bzw. für den Fall, dass genau zwei Temperaturfühler verwendet werden:

$$T_{ist} = (T_1 + T_2)\,/\,2$$

Zur Temperaturregelung in dem gekühlten Innenraum wird $T_{ist}$ auf eine Solltemperatur geregelt, die vorzugsweise nutzerseitig einstellbar ist, wozu entsprechende Einstellmittel vorhanden sein können.

Dieser Temperaturregelung ist die Regelung überlagert, die die Temperaturschichtung, d.h. den Temperaturgradienten in vertikaler Richtung so einstellt, dass dieser einen Grenzwert ($\Delta T_{soll}$) nicht übersteigt, d.h. für die beiden in Figur 1 gezeigten Temperaturfühler $T_1$ und $T_n$ gilt dann:

$$\left|\,T_1 - T_n\,\right| \leq \Delta T_{soll}$$

Wird festgestellt, dass diese Bedingung für die Temperaturschichtung nicht erfüllt ist, steuert der Regler das oder die Peltierelemente so an, dass der Betrag der Differenz der gemessenen Temperaturwerte nicht größer ist als der genannte Grenzwert.

Wie bereits oben ausgeführt, kann der Grenzwert $\Delta T_{soll}$ auch den Wert Null betragen, was bedeutet, dass keine Temperaturschichtung gewünscht ist.

Denkbar ist es, dass das erfindungsgemäße Gerät Einstellmittel aufweist, mittels derer ein Nutzer oder herstellerseitig die Größe $\Delta T_{soll}$ eingestellt werden kann.

Eine mögliche Schaltungsvariante ist in Figur 2a) dargestellt. Über den einfachen Spannungsteiler erhält man zwar eine gewisse Verlustleistung, die Temperaturschichtung kann auf diese Weise jedoch relativ einfach beeinflusst werden. Die Bezugszeichen P1 und P2 kennzeichnen die Peltierelemente, von denen sich eines in dem Bereich A und eines in dem Bereich B befindet.

Figur 2b) zeigt die Regelstrecke zur Regelung der Temperaturdifferenz in vertikaler Richtung des Geräteinnenraums. Die Sollgröße $\Delta T_{soll}$ wird zusammen mit der tatsächlichen Abweichung $|T_1 - T_2|$ dem Regler zugeführt. Aus dem Vergleich ergibt sich die Stellgröße R(t), die auf die Regelstrecke einwirkt, indem die Peltierelemente P1 und P2 entsprechend angesteuert werden.

Anstelle der Peltierelemente in den Bereichen A und B können auch weitere Peltierelemente oder sonstige Kühl- oder Heizelemente verwendet werden, um den Innenraum in der gewünschten Weise zu temperieren.

**Patentansprüche**

1. Temperiertes Gerät, insbesondere Kühl- und/oder Gefriergerät, mit einem Gerätekorpus und mit einem Verschlusselement, mittels dessen der temperierte Innenraum des Gerätes verschließbar ist,
**dadurch gekennzeichnet,**
**dass** in dem temperierten Innenraum wenigstens ein erster und ein zweiter Temperaturfühler zur Erfassung eines ersten und eines zweiten Temperaturwertes angeordnet sind, die sich in unterschiedlichen Höhenpositionen befinden und dass ein Regler vorgesehen ist, der ausgebildet ist, den Istwert der Temperaturdifferenz zwischen den beiden Temperaturwerten mit einem Sollwert zu vergleichen, wobei in einem oberen Bereich und/oder in einem relativ dazu unten angeordneten Bereich des Gerätes mindestens ein Peltierelement angeordnet ist, das mit dem Regler in Verbindung steht, wobei der Regler derart ausgebildet ist, dass der Regler das oder die Peltierelemente so ansteuert, dass die Abweichung zwischen Ist- und Sollwert gleich Null ist oder einen Grenzwert nicht übersteigt.

2. Temperiertes Gerät nach Anspruch 1, **dadurch gekennzeichnet, dass** ein Peltierelement im unteren Drittel des Gerätes angeordnet ist und/oder ein Peltierelement im oberen Drittel des Gerätes angeordnet ist.

3. Temperiertes Gerät nach Anspruch 1 oder 2, **dadurch gekennzeichnet, dass** ein Peltierelement im Boden des Gerätes angeordnet ist und/oder dass ein Peltierelement in der Decke des Gerätes angeordnet ist.

4. Temperiertes Gerät nach einem der vorhergehenden Ansprüche, **dadurch gekennzeichnet**, kein Ventilator zur Luftbewegung in dem temperierten Innenraum vorgesehen ist.

5. Temperiertes Gerät nach einem der vorhergehenden Ansprüche, **dadurch gekennzeichnet, dass** es sich um ein Kühl- und/oder Gefriergerät handelt und dass der Regler derart ausgebildet ist, dass das im oberen Bereich angeordnete Peltierelement aktiviert wird, wenn die Differenz zwischen Ist- und Sollwert einen Grenzwert übersteigt.

6. Temperiertes Gerät nach einem der vorhergehenden Ansprüche, **dadurch gekennzeichnet, dass** es sich um beheiztes Gerät, insbesondere um einen Wärmeschrank handelt und dass der Regler derart ausgebildet ist, dass das im unteren Bereich angeordnete Peltierelement aktiviert wird, wenn die Differenz zwischen Ist- und Sollwert einen Grenzwert übersteigt.

7. Temperiertes Gerät nach einem der vorhergehenden Ansprüche, **dadurch gekennzeichnet, dass** außer dem oder den Peltierelementen keine weiteren Vorrichtungen zur Kühlung und/oder Erwärmung des gekühlten Innenraums vorgesehen sind.

8. Temperiertes Gerät nach einem der Ansprüche 1 bis 6, **dadurch gekennzeichnet, dass** außer dem oder den genannten Peltierelementen weitere Vorrichtungen zur Kühlung und/oder Erwärmung des gekühlten Innenraums vorgesehen sind.

9. Temperiertes Gerät nach einem der vorhergehenden Ansprüche, **dadurch gekennzeichnet, dass** der Regler ausgeführt ist, aus den durch die Temperaturfühler erfassten Temperaturwerten einen Mittelwert zu bilden und diesen mit einer Solltemperatur zu vergleichen und basierend auf der Differenz zwischen dem Mittelwert und der Solltemperatur die Temperaturregelung des temperierten Innenraums vornimmt.

10. Temperiertes Gerät nach einem der vorhergehenden Ansprüche, **dadurch gekennzeichnet, dass** das oder die Peltierelemente mit einem Wärmetauscher in Verbindung stehen, der seinerseits mit dem temperierten Innenraum in Verbindung steht, um eine möglichst große Wärmeübertragungsfläche bereitzustellen.

Fig. 1

Fig. 2

a)

b)

EP 3 477 234 A1

**Europäisches Patentamt**
**European Patent Office**
**Office européen des brevets**

# EUROPÄISCHER RECHERCHENBERICHT

Nummer der Anmeldung

EP 18 20 1990

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2016/178264 A1 (OBBARD RACHEL W [US] ET AL) 23. Juni 2016 (2016-06-23) <br> * Absatz [0020] - Absatz [0022]; Ansprüche 7,9,19,20,21; Abbildungen 1,4,5 * <br> * Absatz [0028]; Abbildung 2 * <br> ----- | 1-10 | INV. <br> F25D31/00 <br> F25B21/02 <br> C12M1/00 <br> B01L7/00 |
| X | US 2009/139248 A1 (CRUMLIN ETHAN J [US] ET AL) 4. Juni 2009 (2009-06-04) <br> * Absatz [0032] - Absatz [0033]; Abbildung 2 * <br> ----- | 1-3,7,9, 10 | |
| X | CH 709 751 A2 (V ZUG AG [CH]) 31. Dezember 2015 (2015-12-31) <br> * Absatz [0021] - Absatz [0023]; Abbildungen 1,2 * <br> ----- | 1,2,6-8 | |
| X | JP 2016 090131 A (MITSUBISHI ELECTRIC CORP) 23. Mai 2016 (2016-05-23) <br> * Abbildungen 2,6 * <br> ----- | 1-3,7,8 | |
| A | DE 11 34 395 B (SIEMENS ELEKTROGERAETE GMBH) 9. August 1962 (1962-08-09) <br> * Abbildung 1 * <br> ----- | 4-7 | **RECHERCHIERTE SACHGEBIETE (IPC)** <br> F25D <br> F25B <br> C12M <br> B01L |
| A | DE 10 2015 006583 A1 (LIEBHERR HAUSGERÄTE LIENZ GMBH [AT]; LIEBHERR HAUSGERÄTE OCHSENHAUSEN) 17. Dezember 2015 (2015-12-17) <br> * Absätze [0046], [0066]; Ansprüche 3,13; Abbildung 1 * <br> ----- | 4-7 | |

Der vorliegende Recherchenbericht wurde für alle Patentansprüche erstellt

| Recherchenort | Abschlußdatum der Recherche | Prüfer |
|---|---|---|
| Den Haag | 14. Februar 2019 | Kuljis, Bruno |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes
    Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 20 1990

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-02-2019

| Im Recherchenbericht angeführtes Patentdokument | Datum der Veröffentlichung | Mitglied(er) der Patentfamilie | Datum der Veröffentlichung |
|---|---|---|---|
| US 2016178264 A1 | 23-06-2016 | KEINE | |
| US 2009139248 A1 | 04-06-2009 | KEINE | |
| CH 709751 A2 | 31-12-2015 | AU 2016250363 A1<br>CH 709751 A2<br>CN 106679272 A<br>EP 3171105 A1 | 25-05-2017<br>31-12-2015<br>17-05-2017<br>24-05-2017 |
| JP 2016090131 A | 23-05-2016 | KEINE | |
| DE 1134395 B | 09-08-1962 | KEINE | |
| DE 102015006583 A1 | 17-12-2015 | CN 106461292 A<br>DE 102015006583 A1<br>EP 3155329 A1<br>US 2017131000 A1<br>WO 2015192952 A1 | 22-02-2017<br>17-12-2015<br>19-04-2017<br>11-05-2017<br>23-12-2015 |

EPO FORM P0461

Für nähere Einzelheiten zu diesem Anhang : siehe Amtsblatt des Europäischen Patentamts, Nr.12/82